# EUROPEAN PATENT APPLICATION

(11) **EP 4 260 886 A1**
(43) Date of publication of application: **18.10.2023**
(21) Application number: 22168319.6
(22) Date of filing: 14.04.2022
(51) Int. Cl.: A61M 5/142, A61M 5/32

(54) **PATCH-LIKE MEDICAL DEVICE WITH MOVABLE NEEDLE PROTECTION SLEEVE**

(71) Applicant: PharmaSens AG, 4105 Biel-Benken (CH)
(72) Inventor: Lodico, Tamara, 5014 Gretzenbach (CH); Teutsch, David, 3036 Detligen (CH); Schranz, Samuel, 3252 Worben (CH); Both, Marcel, 3038 Kirchlindach (CH)
(74) Representative: Detken, Andreas

(57) **Abstract**

A patch-like medical device comprises a base (10) having a contact surface (11) configured to be directed towards a patient's skin, a needle (12) fixedly positioned relative to the base (10), a needle protection sleeve (20) movable from a first position to a second position, a rotating sleeve (30) being in engagement with the needle protection sleeve (20) such that a helical movement of the rotating sleeve (30) with respect to the base (10) causes the needle protection sleeve to move from the first position to the second position, and a rotation lock sleeve (40) being in engagement with the needle protection sleeve (20) such that the needle protection sleeve (20) is prevented from rotating with respect to the base (10) when moving from the first position to the second position.

## Description

### TECHNICAL FIELD

The present invention relates to a patch-like medical device configured to be applied to a patient's skin, the patch-like medical device having a movable needle protection sleeve.

### PRIOR ART

Certain medical conditions, such as for instance diabetes, require regular administration of a liquid substance, such as insulin, into a patient's body through the patient's skin. To facilitate administration, patch-like medical devices are known, which are configured to be applied to the patient's skin and preferably to be worn by the patient over an extended period of time. Such patch-like devices generally comprise a hollow needle (cannula) configured to pierce the patient's skin and through which needle the liquid substance is being delivered into the patient's body. In particular if the liquid substance is to be administered slowly and/or regularly over an extended period of time, the needle is advantageously connected to a delivery system comprising an automatic pump mechanism. In order to be able to pierce the patient's skin when the patch-like medical device is applied to the body, the needle preferably has a sharp needle tip which protrudes from said contact surface of the patch-like medical device. However, before the patch-like medical device is applied to the patient, this needle tip needs to be protected from damage, and any person handling the patch-like medical device prior to use needs to be protected from accidently getting injured by the protruding needle tip.

US5931814A discloses a an injection device having a casing containing an active substance reservoir, a cannula communicating with the reservoir, a device for inserting the cannula, and pump means for discharging the reservoir contents through the cannula. The cannula is fixed relative to the casing and projects beyond the underside of the casing to the depth required for injection. The cannula is surrounded by a protective element which is moved by a spring drive from a first end position, in which the protective device projects beyond the underside of the casing and beyond the cannula, to a second end position in which the protective device does not project beyond the underside of the casing. In particular, a cylindrical protective sleeve is disclosed, which is dimensioned and disposed so that in the inoperative position it projects sufficiently beyond the cannula to prevent accidental contact with the cannula tip. To insert the cannula, the protective sleeve is retracted by spring force into the casing.

Since the needle has to protrude beyond the underside of the casing by a length which is sufficient to achieve a required injection depth, the protective sleeve has to extend over said length as well to fulfill its protective purpose. As a consequence, said length defines a minimum thickness the casing needs to have in order to provide sufficient space for the protective sleeve to be fully received within the casing when the protective sleeve is retracted.

For the patient however, an increase of the casing thickness negatively impacts the comfort.

### SUMMARY OF THE INVENTION

It is an object of the present invention to provide a patch-like medical device with a needle protection mechanism that allows the patient's skin to be pierced easily, while being compact and thus allowing for the patch-like medical device to be slim, which in turn enhances the patient's comfort.

This object is achieved by a patch-like medical device according to claim 1. Further embodiments of the invention are laid down in the dependent claims.

The present invention provides a patch-like medical device comprising:
a base having a contact surface configured to be directed towards to a patient's skin;
a needle fixedly positioned relative to the base, the needle having a needle tip which protrudes from the contact surface and which is configured for piercing the patient's skin, and
a needle protection sleeve movable along a central axis from a first position to a second position, the needle protection sleeve extending beyond the needle tip to prevent the needle tip from piercing the patient's skin in the first position, and exposing the needle tip to enable piercing the patient's skin in the second position.

The patch-like medical device of the present invention further comprises a rotating sleeve movable along the central axis, the rotating sleeve being in engagement with the needle protection sleeve such that a helical movement of the rotating sleeve with respect to the base causes the needle protection sleeve to move along the central axis from the first position to the second position.

Furthermore, patch-like medical device of the present invention comprises a rotation lock sleeve movable along the central axis while being rotationally fixed with respect to the base, the rotation lock sleeve being in engagement with the needle protection sleeve such that the needle protection sleeve is prevented from rotating with respect to the base when the needle protection sleeve moves from the first position to the second position.

The term "needle" as used herein is equivalent to the term "infusion needle" or "cannula" and refers to any elongated sharp element configured to pierce the patient's skin and preferably having a lumen for delivering a liquid substance, in particular insulin, through the patient's skin.

The central axis preferably coincides with an injection axis (longitudinal axis) defined by the needle and is preferably perpendicular to the contact surface. In the present context, the term "distal" refers to a direction parallel to the central axis leading away from the contact surface, i.e. towards the patient's skin when the patch-like medical device is applied as intended. The term "proximal" refers to a direction which is 180° opposite to the distal direction, i.e. leading away from the patient's skin when the patch-like medical device is applied as intended. According to this definition, the needle protection sleeve moves in proximal direction when moving from the first position to the second positon.

Preferably, the needle protection sleeve, the rotating sleeve and the rotation lock sleeve are concentrically arranged around the central axis.

In the first position, the rotating sleeve and/or the rotation lock sleeve preferably at least partially protrudes from the contact surface in the distal direction, and the needle protection sleeve preferably telescopically protrudes beyond the rotating sleeve and the rotation lock sleeve.

The contact surface may comprise an adhesive layer or suction elements for direct adhesion to the patient's skin. Alternatively, the contact surface may be configured to be attached to an adhesive sheet, wherein the sheet is in turn configured to be in direct contact with the patient's skin. In such a case, the needle protection sleeve, the rotating sleeve and the rotation lock sleeve are preferably not covered by said sheet, i.e. the sheet is preferably ring-shaped with a central hole arranged such that, in the first position, the sleeves may protrude from the contact surface through said hole.

Having the rotating sleeve perform a helical movement while being in engagement with the needle protection sleeve and having a rotation lock sleeve being in engagement with the needle protection sleeve enables a telescopic interaction between the rotating sleeve, the needle protection sleeve and the rotation lock sleeve and thus provides a compact needle protection solution.

The needle protection sleeve and the rotating sleeve are preferably in a thread-like engagement with each other.

In the present context, a "thread-like engagement" refers to a mechanical engagement between a first part and a second part that causes a screwing motion, i.e. a combined axial and rotational movement, of the first part relative to the second part. In particular, a thread-like engagement may involve a thread, for instance in form of a helical groove, on the first part and a thread or thread portions, for instance in form of helical ribs, on the second part, the thread portions of the second part being configured to engage with said thread of the first part. The threads and the thread portions mentioned in this disclosure may in particular be square threads, i.e. have a square profile.

The rotating sleeve preferably at least partially protrudes from the contact surface when the needle protection sleeve is in the first position, wherein the rotating sleeve axially retracts towards the contact surface, i.e. in proximal direction, along the central axis during its helical movement, and wherein the needle protection sleeve is configured to axially move relative to the rotating sleeve along the central axis during the helical movement of the rotating sleeve. The rotation lock sleeve may be entrained to axially retract towards the contact surface by the rotating sleeve or by the needle protection sleeve during the helical movement of the rotating sleeve.

In the second position, the needle protection sleeve, the rotating sleeve and the rotation lock sleeve may each be at least partially, preferably fully, retracted inside the patch-like medical device on a side that faces away from the contact surface. In the present context, "fully retracted" means that neither the needle protection sleeve nor the rotating sleeve nor the rotation lock sleeve are protruding from the contact surface, which represents a preferred state for the second position in terms of comfort for the patient.

Due to the telescopic interaction between the rotating sleeve, the needle protection sleeve and the rotation lock sleeve, each of these sleeves may have a length in axial direction with respect to the central axis that is shorter than a needle protrusion length by which the needle protrudes from the contact surface towards the patient's skin, the sleeves thus taking up less space within the patch-like medical device when retracted, thereby allowing the patch-like medical device to be slimmer in axial direction and thus more comfortable for the patient.

The base may form a housing lower part and the patch-like medical device may comprise a housing upper part connected to the base. In some embodiments, the base may simply have a through-hole, and the sleeves may be received in a cavity formed between the base and the housing upper part. Preferably however, the base may define a recess on the side that faces away from the contact surface for receiving the sleeves. The recess may be delimited in axial direction by a top wall. In radial direction, the recess may be delimited by a side wall, the side wall being preferably cylindrical. The top wall may have bore in which the needle is held. An axial protrusion may be present around the needle, the axial protrusion extending distally from the top wall into the recess. The axial protrusion may act to provide the needle with added stability against lateral tilting and bending movements. In other embodiments, the needle may be held by any other type of needle holding structure that is fixedly connected to the base. The medical patch-like device preferably comprises a reservoir configured to contain a liquid substance, preferably insulin. The needle may be fluidically connected to the reservoir. The medical patch-like device may comprise a pump mechanism causing the liquid substance to be pumped from the reservoir through the needle. The pump mechanism and the reservoir are preferably arranged on the side of the base facing away from the contact surface and are ideally protected by the housing upper part. The housing upper part may be configured to be easily removable from the base to enable a refill or exchange of the reservoir. Alternatively, the base and the housing upper part may be permanently sealed. In the patch-like medical device disclosed here, the needle is fixedly positioned with respect to the contact surface of the base, which provides the advantage that the patient may comfortably move while wearing the patch-like medical device without causing a flow of the liquid substance to be inadvertently interrupted by an accidental movement of the needle.

In particular, the needle protection sleeve may have, at a distal end, a protection sleeve front wall defining a protection sleeve contact surface, which is in contact with the patient's skin when the patch-like medical device is applied to the patient. The protection sleeve contact surface may comprise an adhesive layer or suction elements to help securing the patch-like medical device to the patient's skin.

The protection sleeve front wall may have a needle hole, through which needle hole the needle tip protrudes when the needle protection sleeve is in the second position. Preventing the needle protection sleeve from rotating with respect to the base thus ensures that the patient's skin is not getting twisted while the needle protection sleeve is moving from the first position to the second position. When first applying the medical patch-like device to the patient's skin, the patient's skin may be slightly pushed in by the needle protection sleeve. While the needle protection sleeve is moving from the first position to the second position, the patient's skin may resiliently move towards the needle tip following the needle protection sleeve, thus leading to an easy and nearly pain-free insertion of the needle tip into the patient's skin.

In some embodiments, the needle protection sleeve may be at least partially received radially inside the rotating sleeve, and the rotating sleeve and the sidewall delimiting the recess defined by the base may be in a thread-like engagement with each other. Preferably, the thread-like engagement of the protection sleeve with the rotating sleeve exhibits a handedness that is opposite to the handedness of the thread-like engagement of the rotating sleeve with the side wall that delimits the recess.

More specifically, the side wall that delimits the recess may comprise an internal thread (i.e., a helical groove), and the rotating sleeve may comprise at least one external thread portion (i.e., a helical rib) configured to engage with said internal thread of the recess. In particular, the internal thread of the recess may be a triple-start thread and the rotating sleeve may comprise three external thread portions, to ensure good stability against tilting movements of the rotating sleeve even if the thread portions are short (e.g., less than 120° in a circumferential direction). Alternatively, the rotating sleeve may have an outer surface comprising a helical groove extending helically about the central axis, and the recess may comprise a thread portion (helical rib) protruding radially inwards towards the central axis, the thread portion being in engagement with the helical groove on the outer surface of the rotating sleeve.

In other embodiments, the rotating sleeve may be at least partially received radially inside the needle protection sleeve. In such embodiments, it is advantageous if the base comprises a central guide element, preferably configured as a hollow cylindrical element, arranged within the recess, the central guide element being extending distally from the top wall into the recess along the central axis. The central guide element may be at least partially received radially inside the rotating sleeve, and the rotating sleeve and the central guide element may be in a thread-like engagement with each other.

In particular, the central guide element may have an outer surface comprising a helical groove extending helically about the central axis, and the rotating sleeve may have at least one thread portion (helical rib) protruding radially inwards towards the central axis configured to engage with the helical groove on the outer surface of the central guide element.

In some embodiments, the rotating sleeve may comprise an internal thread and the needle protection sleeve may comprise at least one external thread portion engaging with said internal thread of the rotating sleeve. In particular, the rotating sleeve may be a triple-start thread and the needle protection sleeve may comprise three external thread portions.

In other embodiments, the needle protection sleeve may comprise an internal thread and the rotating sleeve may comprise at least one external thread portion engaging with said internal thread of the needle protection sleeve. In particular, the internal thread of the needle protection sleeve may be a triple-start thread and the rotating sleeve may comprise three external thread portions.

One of the needle protection sleeve and the rotation lock sleeve may comprise a longitudinal groove extending parallel to the central axis, wherein the other one of the needle protection sleeve and the rotation lock sleeve may comprise a guide cam configured to slide in said longitudinal groove such that the protection sleeve is prevented from rotating with respect to the base when moving from the first position to the second position.

As a resulting effect, the rotation lock sleeve prevents the needle protection sleeve from rotating with respect to the base.

In particular, in some embodiments, the needle protection sleeve may have an outer surface comprising a longitudinal groove, wherein the longitudinal groove extends parallel to the central axis and the rotation lock sleeve may comprise a guide cam being in engagement with said longitudinal groove of the needle protection sleeve. In a preferred version, the needle protection sleeve has an outer surface comprising preferably three longitudinal grooves and the rotation lock sleeve comprises preferably three guide cams, wherein each guide cam is in engagement with one longitudinal groove.

Alternatively, in other embodiments, the rotation lock sleeve may have an inner surface comprising a longitudinal groove, wherein the longitudinal groove extends parallel to the central axis and the needle protection sleeve may comprise a guide cam being in engagement with the longitudinal groove of the rotation lock sleeve. In particular, the inner surface of the rotation lock may comprise at least three longitudinal grooves and the needle protection sleeve may comprise at least three guide cams equidistantly spaced with respect to a circumferential direction to provide sufficient stability.

The patch-like medical device may further comprise a fixation element that is fixedly attached to the base and is in engagement with the rotation lock sleeve such that the rotation lock sleeve is prevented from rotating relative to the base, wherein the fixation element is preferably configured as a fixation ring

The fixation element may comprise a notch, and the rotation lock sleeve may have an outer surface comprising a ridge extending parallel to the movement axis and being in engagement with the notch of the fixation ring. Preferably, the fixation ring comprises three or four notches, ideally equally distributed along a circumference of the fixation ring for better stability, and the rotation lock sleeve accordingly comprises three or four ridges.

Furthermore, the fixation element may act as a stop element against which the rotation lock sleeve may axially abut in distal direction when the needle protection sleeve is in the first position, thus preventing the rotation lock sleeve and the rotating sleeve from disengaging from the base. The fixation element may be a separate piece attached to the base or may be made in one piece with the base. In case the fixation element is a separate piece, it may be attached to the casing by soldering, screwing or gluing.

The patch-like medical device may further comprise a spring configured to drive the helical movement of the rotating sleeve, the spring being pre-loaded when the needle protection sleeve is in the first position, wherein the spring at least partially relaxes when the needle protection sleeve moves from the first position to the second position.

In particular, the spring may be a leg spring or a torsion spring having a first end and a second end, the first end being connected to the rotation lock sleeve and the second end being connected to the rotating sleeve, thereby exerting a torsion force on the rotating sleeve and urging it to carry out the helical movement. The rotation lock sleeve may have a first bore configured to receive the first end of the spring and the rotating sleeve may have a second bore configured to receive the second end of the spring.

Alternatively, the spring may also be a tension spring. In particular, a tension force may be exerted on the rotating sleeve by the tension spring, pulling the rotating sleeve towards the base, i.e. in proximal direction, thereby urging it to carry out the helical movement, for example by forcing it to follow the helical grooves on the outer surface of the central guide element in embodiments that comprise said central guide element. In such a case, the thread-like engagement of the rotating sleeve and the central guide element preferably has a thread pitch that is sufficiently steep such that said force is sufficient to cause the helical movement. In some embodiments, the tension spring may have a first end being tightly wrapped around a distal protrusion of the base, wherein said distal protrusion is preferably arranged radially inside the cylindrical element and is preferably extending distally along the central axis towards a central opening of the rotating sleeve, and a second end being tightly wrapped around a protrusion of the rotating sleeve, wherein the protrusion of the rotating sleeve is preferably extending proximally along the central axis towards the protrusion of the base.

The helical movement of the rotating sleeve may alternatively also be driven by a force applied to the needle protection sleeve in proximal direction towards the base. In particular, said force may be exerted by the patient's skin. In such a case, the thread-like engagement of the needle protection sleeve and the rotating sleeve preferably has a thread pitch that is sufficiently steep such that said force is sufficient to cause the helical movement.

The patch-like medical device may further comprise a locking mechanism having a locked state in which the locking mechanism prevents the rotating sleeve from moving when the needle protection sleeve is in the first position, and an unlocked state in which the locking mechanism allows the rotating sleeve to carry out the helical movement causing the needle protection sleeve to move from the first position to the second position.

Furthermore, the patch-like medical device may comprise a trigger mechanism operable by the patient, wherein the trigger mechanism is configured to switch the locking mechanism from the locked state to the unlocked state.

The locking mechanism may comprise a latch pivotably connected to the base, the latch being engaged with the rotating sleeve in the locked state to prevent the rotating sleeve from moving and disengaged from the rotating sleeve in the unlocked state to allow the rotating sleeve to carry out the helical movement. In particular, the latch may comprise a tip which engages with an indentation located in a rim of the rotating sleeve. The latch may further comprise a shaft extending through the base parallel to the central axis to reach the side of the base opposite to the contact surface.

Furthermore, the locking mechanism may comprise a lever element being connected to the latch, preferably to the shaft of the latch on the side of the base opposite to the contact surface, for facilitating control of the latch, wherein the lever element is preferably movable between a first lever element position in which the lever element prevents the latch from disengaging from the rotating sleeve, and a second lever element position in which the lever element allows the latch to disengage from the rotating sleeve. The lever element may be shaped in a variety of different ways. In some embodiments, in may be an injection molded part. In other embodiments, it may be made of a sheet metal piece, which may be resiliently bendable. The lever element may have an opening on a first end, in which the end of the shaft of the latch may be fixed. It may have a second end which is, in the first lever element position, biased in distal direction towards the base, in particular bent to abut against a stopper element, the stopper element being attached to the base. In said first lever element position, which corresponds to the locked state of the locking mechanism, the lever element may prevent the latch from disengaging from the rotating sleeve by preventing rotation of the shaft, which is fixedly connected to the first end of the lever element. In the second lever element position, the lever element preferably allows the latch to disengage from the rotating sleeve.

In addition, the locking mechanism may comprise a clamp element configured for clamping the lever element to prevent inadvertent motion of the lever element and the latch, wherein the clamp element is preferably movable between a first clamp position in which the clamp element fixes the lever element with respect to the base in the first lever element position, and a second clamp position in which the clamp element releases the lever element and allows the lever element to move from the first lever element position to the second lever element position. With the lever element moving from the first lever element position to the second lever element position, the latch may be able to pivot and thus to disengage from the rotating sleeve. The rotating sleeve may then be free to perform the helical movement. In cases where a spring is driving said helical movement, the spring is preferably pre-loaded such that the rotating sleeve exerts a force on the latch when the needle protection sleeve is in the first position and the locking mechanism is in the locked state. This force may urge the latch to pivot radially outwards and release the rotating sleeve, but clamping of the lever element in the first lever element position by the clamp element may prevent the latch from pivoting.

In case the fixation element is a fixation ring, the latter preferably covers the latch, such that that latch is hidden and is not in contact with the patient's skin when the patch-like medical device is worn by the patient. In particular, the contact surface may have a recessed area in which the latch may be arranged. The recessed area is then preferably configured to receive the fixation ring such that the fixation ring is flush with the contact surface and covers the latch when attached to the base. In case the fixation element is made in one piece with the base, the latch is preferably hidden within the base.

The trigger mechanism may comprise control circuitry and an actuation mechanism configured to mechanically actuate the clamp element upon receiving an electrical signal from the control circuitry, causing the clamp element to move from the first clamp position to the second clamp position so as to release the lever element. The actuation mechanism may comprise a motor-driven threaded shaft, which is preferably configured to rotate upon receiving a signal from the control circuitry, and a transmission piece that may comprise a fork-like part, wherein the threaded shaft is preferably in a gear-like engagement with the transmission piece. The actuation mechanism may further comprise a pivot arm having a spherically shaped top end that is embraced by the fork-like part of the transmission piece. Upon receiving a signal from the control circuitry, the threaded shaft may starts to rotate, thereby moving the transmission piece from a first position to a second position. Preferably, the fork-like part of the transmission piece thereby drags along the spherically shaped top end of the pivot arm, causing the pivot arm to pivot from a first pivot position to a second pivot position. The actuation mechanism may further comprise a wire arrangement which may be at least partially wrapped around the sidewall delimiting the recess and which is preferably configured to urge the clamp element to move in proximal direction from the first clamp position to the second clamp position. The pivot arm may have a nose-like protrusion, which in the first pivot position pushes the clamp element in distal direction (and thereby prevents it from moving) against a force exerted on the clamp element by the resilient wire arrangement. When the pivot arm is being moved to the second pivot position, the clamp element preferably moves in proximal direction, being preferably linearly guided by a guiding structure that may be arranged on the base. As the clamp element moves in proximal direction, it preferably releases the lever element, which then may pivot from the first lever element position to the second lever element position as the latch is pushed radially outward by the rotating sleeve that is urged to rotate by the pre-loaded spring.

In order to activate the trigger mechanism, a button, in particular at least two buttons, preferably three buttons, may have to be pushed simultaneously by a user. The control circuitry may be configured to determine a switching state of the buttons and to output the electrical signal based on said switching state. Having to push several buttons simultaneously may help to reduce the risk of an unwanted accidental activation. The trigger mechanism may further be connected to the pump mechanism and configured to initiate a flow of the liquid substance from the reservoir through the needle to inject the liquid substance into the patient's body once the needle protection sleeve is in the second position. The flow of the liquid substance may be initiated automatically after the needle protection sleeve has reached the second position. Alternatively, initiating the flow of the liquid substance may require the user to push the same buttons again or may require the user to push at least one additional button separately configured to trigger the pump mechanism.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an exploded view of a patch-like medical device according to a first embodiment of the present invention;
- Fig. 2A: shows a sectional view of the patch-like medical device of Fig. 1 in a first position;
- Fig. 2B: shows a sectional view of the patch-like medical device of Fig. 1 in an intermediate position;
- Fig. 2C: shows a sectional view of the patch-like medical device of Fig. 1 in a second position;
- Fig. 3A: shows a detailed perspective view of a rotating sleeve according to the first embodiment of Fig. 1;
- Fig. 3B: shows a detailed perspective view of a rotation lock sleeve according to the first embodiment of Fig. 1;
- Fig. 3C: shows a detailed perspective view of a needle protection sleeve according to the first embodiment of Fig. 1;
- Fig. 3D: shows a detailed perspective view of fixation ring according to the first embodiment of Fig. 1;
- Fig. 4A: shows a first step of a method of assembly involving components of the first embodiment of Fig. 1;
- Fig. 4B: shows a second step of the method of assembly involving components of the first embodiment of Fig. 1;
- Fig. 4C: shows a third step of the method of assembly involving components of the first embodiment of Fig. 1;
- Fig. 4D: shows the sleeves of the first embodiment of Fig. 1 in a preloaded state, i.e. in the first position, after the third step of the method of assembly;
- Fig. 5: shows an exploded view of a patch-like medical device according to a second embodiment of the present invention;
- Fig. 6: shows a sectional view of the patch-like medical device of Fig. 5 in a first position;
- Fig. 7A: shows a detailed perspective view of a rotating sleeve according to the second embodiment of Fig. 5;
- Fig. 7B: shows a detailed perspective view of a rotation lock sleeve according to the second embodiment of Fig. 5;
- Fig. 7C: shows a detailed perspective view of a needle protection sleeve according to the second embodiment of Fig. 5;
- Fig. 7D: shows a detailed perspective view of fixation ring according to the second embodiment of Fig. 5;
- Fig. 8: illustrates schematically an actuation of the patch-like medical device;
- Fig. 9A: shows a locked state of a first embodiment of a locking mechanism according to the present invention in a perspective view;
- Fig. 9B: shows an unlocked state of the first embodiment of the locking mechanism of Fig. 9A;
- Fig. 9C: shows an enlarged perspective view of a latch and a lever element that are part of the first embodiment of the locking mechanism of Fig. 9A;
- Fig. 10A: shows a top view of the locking mechanism of Fig. 9A in the locked state;
- Fig. 10B: shows a top view of the locking mechanism of Fig. 9A in the unlocked state;
- Fig. 11A: shows a bottom view of the locking mechanism of Fig. 9A in the locked state;
- Fig. 11B: shows a bottom view of the locking mechanism of Fig. 9A in the unlocked state;
- Fig. 12: shows a locked state of a second embodiment of a locking mechanism according to the present invention in a perspective view;
- Fig. 13: shows a locked state of a third embodiment of a locking mechanism according to the present invention in a perspective view, and
- Fig. 14: shows a bottom view of a base where the fixation ring is formed by the base itself.

### DESCRIPTION OF PREFERRED EMBODIMENTS

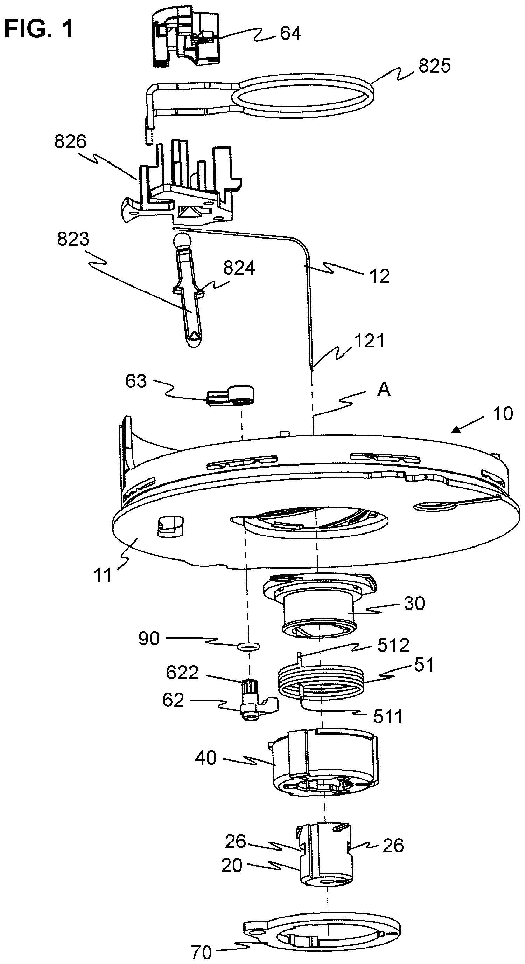

Figures 1 to 4D illustrate a patch-like medical device according to a first embodiment of the present invention.

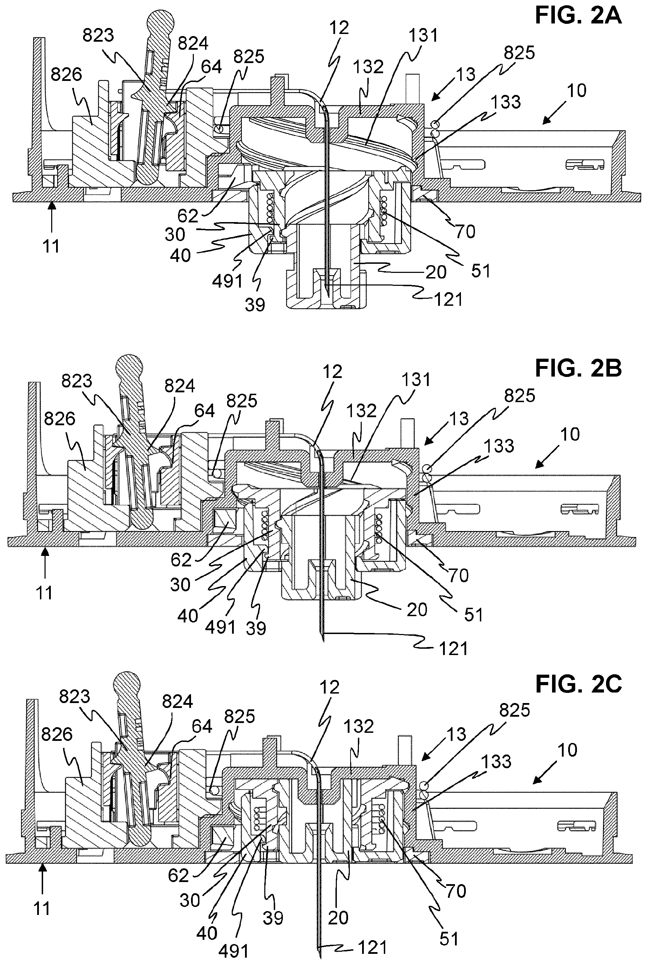

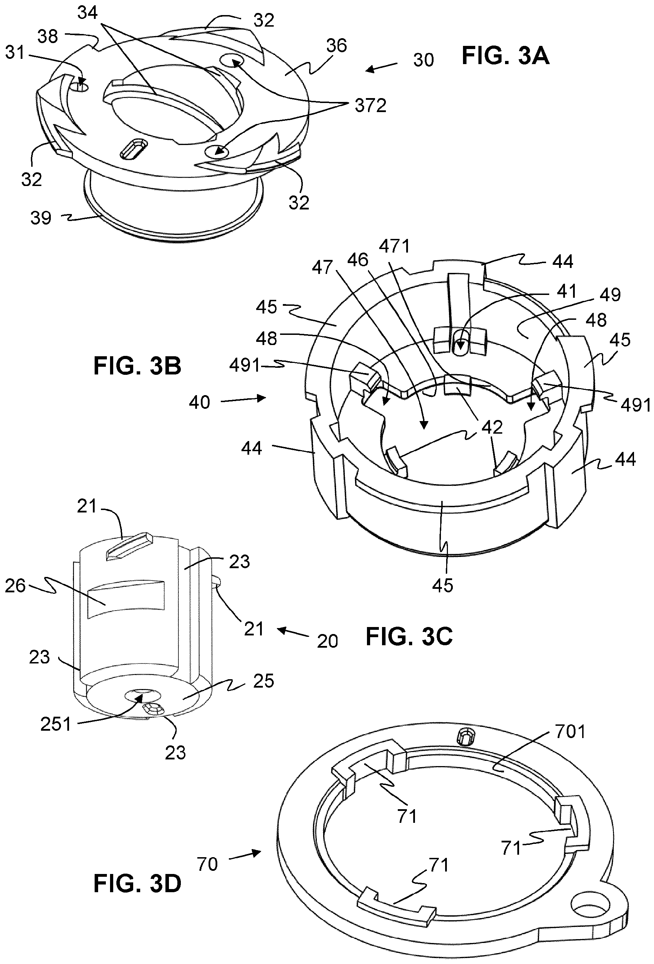

Fig. 1 shows an exploded view comprising individual components, Figs. 2A-C show a sectional view depicting how the individual components are in engagement with each other in this first embodiment of the patch-like medical device, and Figs. 3A-D show perspective detail views of selected individual components. The patch-like medical device comprises a base 10 having a contact surface 11 configured to be directed towards a patient's skin. Said contact surface 11 may comprise an adhesive layer or may be configured to be attached to an adhesive plaster to enable the patch-like medical device to be worn by the patient over an extended period of time. The patch-like medical device further comprises a needle 12 fixedly positioned relative to the base 10. The needle 12 has a needle tip 121 which protrudes from the contact surface 11 and which is configured for piercing the patient's skin. In the embodiments shown here, the needle 12 is a cannula configured to deliver a liquid substance through the patient's skin. The base 10 defines a recess 13 (visible in Fig. 2A-2C) arranged on a side of the base 10 being opposite to the contact surface 11. The recess is delimited in axial direction by a top wall 132. In radial direction, the recess 13 is delimited by a cylindrical side wall 133.

Furthermore, the patch-like medical device comprises a rotating sleeve 30 (shown separately in Fig. 3A) configured to perform a helical movement with respect to the base 10, a rotation lock sleeve 40 (shown separately in Fig. 3B), a needle protection sleeve 20 (shown separately in Fig. 3C) and a fixation element in form of a fixation ring 70 (shown separately in Fig. 3D).

The patch-like medical device shown here further comprises a spring 51. In this first embodiment, the spring 51 is a helical torsion spring capable of exerting a twisting force. The spring 51 has a first end 511 and a second end 512, wherein the rotation lock sleeve 40 has a first bore 41 (as shown in Fig. 3B) in which the first end 511 of the spring is received. The rotating sleeve 30 has a second bore 31 (shown in Fig. 3A) in which the second end 512 of the spring is received.

Prior to use, i.e. prior to piercing the patient's skin, the needle protection sleeve 20 is in a first position, as depicted in Fig. 2A, where it extends beyond the needle tip 121 to protect the latter. In this first position, the rotating sleeve 30 partially protrudes from the contact surface 11 in distal direction. The spring 51 is pre-loaded and in particular exerts a twisting force between the rotating sleeve 30 and the rotation lock sleeve 40. Since the rotation lock sleeve 40 is prevented from rotating by the fixation ring 70, the rotating sleeve 30 is urged to carry out a helical movement in proximal direction. However, to prevent the rotating sleeve 30 from moving prior to use, the rotating sleeve 30 may be held back by a latch 62, which is part of a locking mechanism that will be explained in further detail below. Once the locking mechanism is switched from a locked state to an unlocked state, the latch 62 disengages from the rotating sleeve 30 and allows the latter to rotate due to the twisting force exerted on it by the spring 51. As shown in Fig. 2B, while the spring 51 at least partially relaxes, the rotating sleeve 30 performs the helical movement, i.e. in this case screws itself into the recess 13 while dragging along the rotation lock sleeve 40, which is hooked to a distal rim 39 of the rotating sleeve via three hooks 491. The dragged rotation lock sleeve 40 performs a linear movement in proximal direction, as it is prevented from rotating by the fixation ring 70, which in turn is attached to the base 10. The needle protection sleeve 20, which is prevented from rotating by the rotation lock sleeve 40, linearly move towards the base 10 while effectively getting screwed into the rotating sleeve 30, since the needle protection sleeve 20 is in a thread-like engagement with the rotating sleeve 30. Ultimately, the needle protection sleeve reaches its second position (shown in Fig. 2C), in which the needle tip 121 is exposed. Fig. 2C shows a preferred second position, where all the sleeves are fully retracted inside the recess 13 of the base 10.

The rotating sleeve 30 is in a thread-like engagement with the side wall 133 that delimits the recess 13. The thread-like engagement of the protection sleeve 20 with the rotating sleeve 30 exhibits a handedness that is opposite to the handedness of the thread-like engagement of the rotating sleeve 30 with the side wall 133 that delimits the recess 13. In this first embodiment, said side wall 133 comprises an internal thread 131 in form of a triple-start internal thread (visible in Fig. 2A). The rotating sleeve 30 comprises three external thread portions 32 (shown in detail in Fig. 3A) configured to engage with said triple-start internal thread 131 of the recess 13. The three external thread portions 32 are arranged on a proximal rim 36 of the rotating sleeve 30, the proximal rim 36 extending radially outwards. In the first position, said proximal rim 36 is configured to axially abut in distal direction against rim portions 45 of the rotation lock sleeve 40 (see Fig. 3B), which rim portions 45 also extend radially outwards. The rim portions 45 are in turn configured to axially abut in distal direction against the fixation ring 70 in the first position, which prevents the rotation lock sleeve 40 and hence also the rotating sleeve 30 from disengaging from the patch-like medical device. Two bores 372 are furthermore arranged off-center with respect to the central axis A to enable the insertion of a tool when initially assembling the components, such that the rotating sleeve may be rotated using said tool to thereby pre-tension the spring 51.

In an alternative version of this first embodiment (not shown in the figures), the rotating sleeve 30 may have an outer surface comprising a helical groove extending helically about the central axis A and the recess 13 may comprise a thread portion protruding radially inwards towards the central axis A configured to engage with the helical groove on the outer surface of the rotating sleeve 30.

In this first embodiment, the needle protection sleeve 20 is at least partially received radially inside the rotating sleeve 30, and the needle protection sleeve 20 and the rotating sleeve 30 are in a thread-like engagement with each other. In this particular example, the rotating sleeve 30 comprises an internal thread 34 which is a triple-start internal thread and the needle protection sleeve 20 comprises three external thread portions 21 engaging with said triple-start internal thread 34 of the rotating sleeve 30. As shown in Fig. 3C, the needle protection sleeve 20 has, at a distal end, a protection sleeve front wall 25 defining a protection sleeve contact surface, which is in contact with the patient's skin when the patch-like medical device is applied to the patient. The protection sleeve front wall 25 has a needle hole 251, through which needle hole 251 the needle tip 121 protrudes when the needle protection sleeve 20 is in the second position. The three external thread portions 21 are arranged on an outer surface of the needle protection sleeve 20 at a proximal end of the protection sleeve 20. Furthermore, the outer surface of the needle protection sleeve 20 comprises three longitudinal grooves 23, each longitudinal groove 23 extending parallel to the central axis A from the first end to the second end and being arranged between two of the external thread portions 21 with respect to the circumferential direction. In the embodiment shown here, the three longitudinal grooves 23 have an essentially rectangular cross section, they may however have any other suitable shape. In addition, the outer surface of the needle protection sleeve 20 comprises two indentations 26 extending perpendicularly to the longitudinal grooves and being arranged radially opposite to each other. These indentations 26 are used when initially assembling the components, as will be described below.

The rotation lock sleeve 40 (shown in Fig. 3B) has, at a distal end, a rotation lock sleeve front wall 46, which partially caps the rotation lock sleeve 40. The rotation lock sleeve front wall 46 has an opening 47 centered around the central axis A, through which opening 47 the needle protection sleeve 20 glides when moving from the first position to the second position. To prevent the needle protection sleeve 20 from rotating with respect to the base 10, the rotation lock sleeve 40 comprises three essentially rectangular guide cams 42 protruding radially inward from an edge 471 of the opening 47, wherein each guide cam 42 is in engagement with one of the three longitudinal grooves 23 of the needle protection sleeve 20. Instead of being essentially rectangular, the guide cams 42 may also have any other suitable form. The rotation lock sleeve 40 furthermore comprises three hooks 491 protruding radially inwards from an inner wall 49 of the rotation lock sleeve 40, wherein each hook 491 is arranged between two of the guide cams 42 with respect to the circumferential direction. In this embodiment, the hooks 491 are configured to engage with the distal rim 39 of the rotating sleeve 30 to form a snap-fit connection. In order to facilitate demolding of the hooks 491 during fabrication of the rotation lock sleeve 40, a notch 48 extends radially outwards from the edge 471 of the opening 47 underneath each hook 491. The rim portions 45 described above are arranged at a proximal end of the rotation lock sleeve 40.

Furthermore, the rotation lock sleeve 40 has an outer surface comprising three longitudinal ridges 44 with essentially rectangular profiles, each extending parallel to the central axis A. As shown in Fig. 3D, the fixation ring 70 comprises three notches 71 with essentially rectangular cross-sections extending radially outwards from an inner edge 701 of the fixation ring 70. Each notch 71 is in engagement with one of the ridges 44 of the rotation lock sleeve 40, thereby preventing it from rotating with respect to the base 10. Instead of having essentially rectangular profiles, the longitudinal ridges 44 may have any other suitable profiles, provided that the notches 71 have a corresponding cross-section.

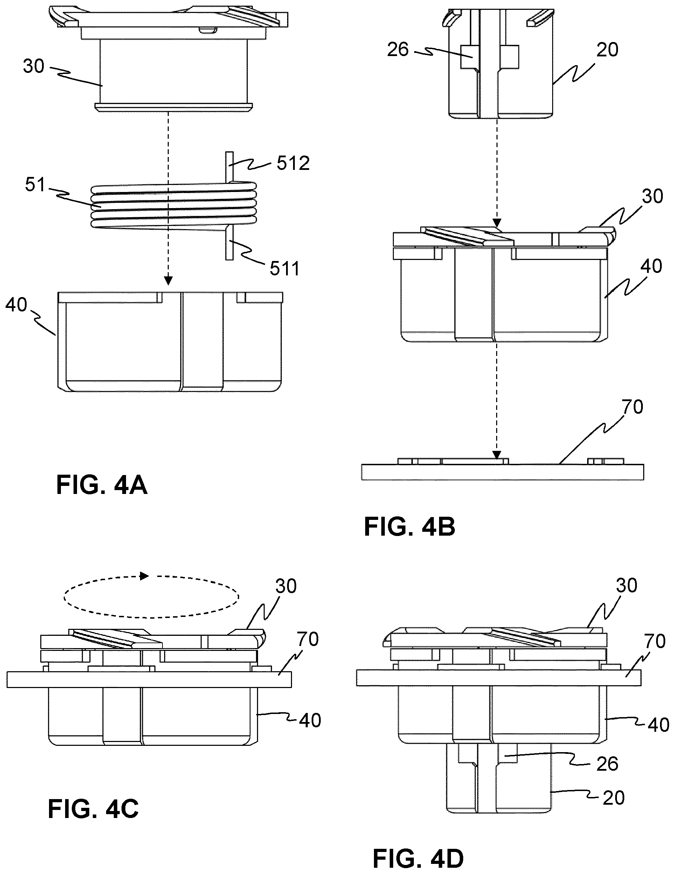

Figs. 4A-4D show how the sleeves of the first embodiment of the present invention may be assembled. In a first step (Fig. 4A), the spring 51 is placed inside the rotation lock sleeve 40 such that first end 511 is received in the first bore 41 (visible in Fig. 3B), and the rotating sleeve 30 is placed inside the rotation lock sleeve 40 such that the second end 512 of the spring 51 is received in the second bore 31 (visible in Fig. 3A). In a second step (Fig. 4B), the needle protection sleeve 20 is inserted into the rotating sleeve 30 from the top, i.e. inserted in distal direction, yielding a pre-assembly comprising the three sleeves and the spring 51, and the pre-assembly is put into the fixation ring 70 from the top. In a third step (Fig. 4C), the rotating sleeve 30 is rotated with respect to the fixation ring 70, which is preferably fixedly mounted in a temporary mount, by using a tool (not shown) inserted in the bores 372 of the rotating sleeve 30 as described above. Rotating the rotating sleeve 30 causes the spring 51 to be pre-loaded and also causes the needle protection sleeve 20 to move in distal direction to finally reach the first position shown in Fig. 4D. Once this first position is reached, the pre-assembly may be kept in this first position by gripping means (not shown) that hold onto the two indentations 26 to prevent the needle protection sleeve 20 from screwing itself back into the rotating sleeve 30 once the tool is removed from the bores 372. The pre-assembly plus the fixation ring 70 may then be mounted into the base 10. The rotating sleeve 30 is then locked by a locking mechanism 60 that will be described in more detail below. Once the rotating sleeve 30 is locked, the gripping means may be removed.

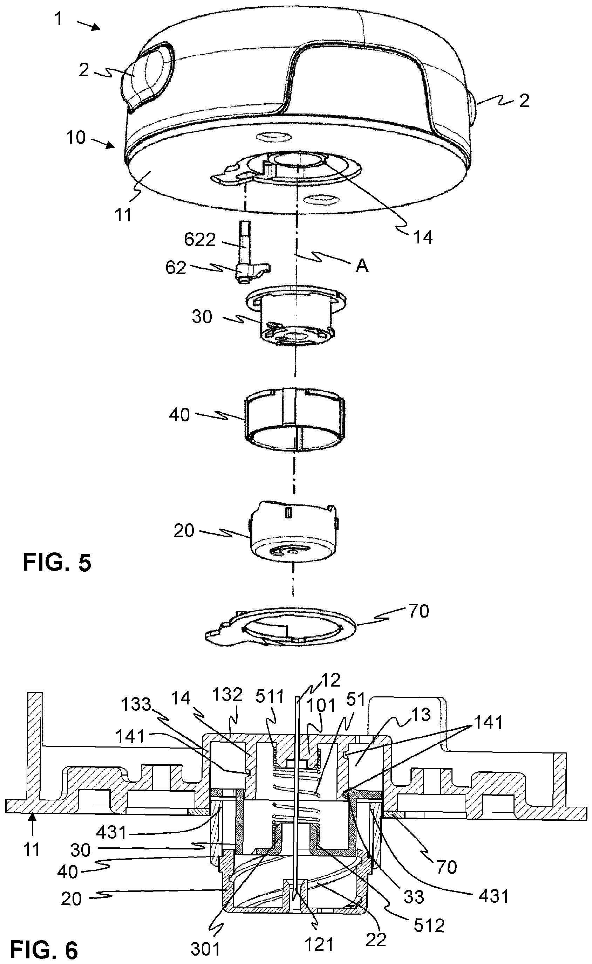

Figs. 5 to 7D illustrate a patch-like medical device according to a second embodiment of the present invention.

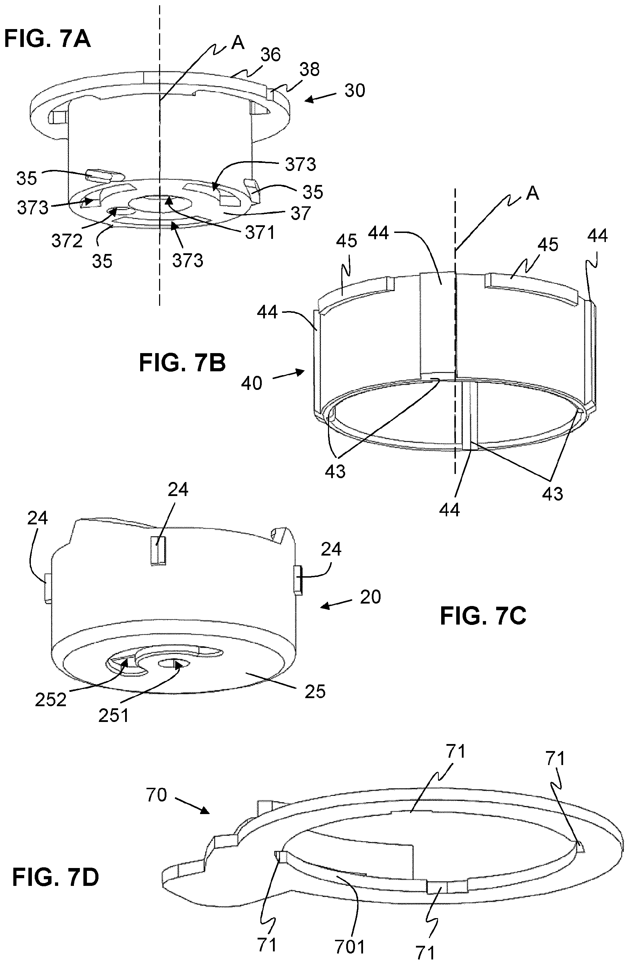

Fig. 5 shows an exploded view comprising individual components, Fig. 6 shows a sectional view depicting how the individual components are in engagement with each other in this second embodiment of the patch-like medical device and Figs. 7A-7D show perspective detail views of selected individual components.

Like the first embodiment described above, the second embodiment comprises a base 10 having a contact surface 11 configured to be applied to a patient's skin, wherein the contact surface 11 may comprise an adhesive layer or may be attached to an adhesive sheet, and a needle (cannula) 12 fixedly positioned relative to the base 10, wherein the needle 12 has a needle tip 121 which protrudes from the contact surface 11 and which is configured for piercing the patient's skin. The base 10 defines a recess 13 arranged on a side of the base 10 being opposite to the contact surface 11, the recess being delimited in axial direction by a top wall 132 and in radial direction by a cylindrical side wall 133.

The patch-like medical device according to the second embodiment comprises a rotating sleeve 30 (shown separately in Fig. 7A), a rotation lock sleeve 40 (shown separately in Fig. 7B), a needle protection sleeve 20 (shown separately in Fig. 7C) and a fixation ring 70 (shown separately in Fig. 7D).

In this second embodiment, the base 10 comprises a central guide element configured as a hollow cylindrical element 14 arranged within the recess 13 and extending in the distal direction along the central axis A, wherein the cylindrical element 14 is at least partially received radially inside the rotating sleeve 30.

In this second embodiment, the spring 51 is a tension spring having a first end 511 being tightly wrapped around a distal protrusion 101 of the base 10 arranged radially inside the cylindrical element 14 and extending distally along the central axis A towards a central opening 371 of the rotating sleeve 30, and a second end 512 being tightly wrapped around a protrusion 301 of the rotating sleeve 30 extending proximally along the central axis A towards the protrusion 101 of the base 10. In the first position (Fig. 6), the spring 51 is pre-loaded and exerts a tension force on the rotating sleeve 30, pulling the rotating sleeve 30 in proximal direction towards the base 10. Similar to the first embodiment described above, once the locking mechanism is switched from a locked state to an unlocked state, the latch 62 disengages from the rotating sleeve 30. The rotating sleeve 30, being pulled by the spring 51, then caries out the helical movement following helical grooves 141 that are arranged on an outer surface of the cylindrical element 14, thereby retracting into the recess 13. The helical movement simultaneously causes the rotating sleeve 30 to screw itself into the needle protection sleeve 20, since the needle protection sleeve 20 is in a thread-like engagement with the rotating sleeve 30 while being prevented from rotating with respect to the base 10, and, in this second embodiment, the rotating sleeve 30 is at least partially received radially inside the needle protection sleeve 20. Here, the thread-like engagement of the protection sleeve 20 with the rotating sleeve 30 exhibits a handedness that is opposite to the handedness of the thread-like engagement between the rotating sleeve 30 and the outer surface of the cylindrical element 14. The needle protection sleeve 20 linearly moves in proximal direction towards the base 10 while being prevented from rotating by longitudinal grooves 43 arranged in an inner surface of the rotation lock sleeve 40 that are in engagement with four guide cams 24 arranged on an outer surface of the needle protection sleeve 20. The needle protection sleeve 20 thereby moves relative to the rotation lock sleeve 40 until it reaches a proximal end of the rotation lock sleeve 40. At the proximal end of the rotation lock sleeve 40, the needle protection sleeve 20 then abuts in proximal direction against end facets 431 of the longitudinal grooves 43, which then causes the needle protection sleeve 20 to drag the rotation lock sleeve 40 along in proximal direction until all sleeves are ideally fully retracted within the recess 13. During retraction, the rotation lock sleeve 40 is furthermore prevented from rotating with respect the base 10 by the fixation ring 70.

In the following, features of this second embodiment are described in more details.

As mentioned above, the rotating sleeve 30 and the cylindrical element 14 are in a thread-like engagement with each other. The cylindrical element 14 has an outer surface comprising three helical grooves 141 (see Fig. 6) extending helically about the central axis A. The rotating sleeve 30 has three thread portions 33 protruding radially inwards towards the central axis A, wherein configured to each thread portion 33 engages with one of the helical grooves 141 on the outer surface of the cylindrical element 14. The rotating sleeve 30 has, at a distal end, a rotating sleeve front wall 37 having a central opening 371, which extends as a protrusion in proximal direction (as visible in the cross-sectional view in Fig. 6) and through which the needle 12 is able to pass. A bore 372 is furthermore arranged off-center with respect to the central axis A to enable the insertion of a tool when initially assembling the components, such that the rotating sleeve may be rotated using said tool to thereby pre-loading the spring 51. In addition, three arched openings 373 are arranged around the central opening 371. In the second position, i.e. when all the sleeves are fully retracted, portions of the cylindrical element 14 comprising the helical grooves 141 are protruding into said arched openings 373 (not directly visible in the figures). This enables the length of the helical grooves 141 to be maximized while keeping a compact configuration. The three thread portions 33 in engagement with the helical grooves 141 are arranged on the inside of the rotating sleeve 30 at a proximal end of the rotating sleeve 30. At a proximal end opposite to the distal end, a rim 36 furthermore extends radially outwards. In the first position, the rim 36 may axially abuts against rim portions 45 of the rotation lock sleeve 40 in the distal direction, which rim portions 45 are arranged at a proximal end of the rotation lock sleeve 40 and also extend radially outwards. The rim portions 45 of the rotation lock sleeve 40 in turn abut axially against the fixation ring 70 in distal direction, which prevents the rotation lock sleeve 40 from disengaging from the base in the first position.

Like in the first embodiment, the needle protection sleeve 20 and the rotating sleeve 30 are in a thread-like engagement with each other. However, in this second embodiment, the rotating sleeve 30 is at least partially received radially inside the needle protection sleeve 20. The needle protection sleeve 20 comprises an internal thread 22 which is a triple-start internal thread and the rotating sleeve 30 comprises three external thread portions 35 engaging with said triple-start internal thread 22 of the needle protection sleeve 20. As shown in Fig. 7C, the needle protection sleeve 20 has, at a distal end, a protection sleeve front wall 25 defining a protection sleeve contact surface, which is in contact with the patient's skin when the patch-like medical device is applied to the patient. Like in the first embodiment, the protection sleeve front wall 25 has a needle hole 251, through which needle hole 251 the needle tip 121 protrudes when the needle protection sleeve 20 is in the second position. Furthermore, in this second embodiment, the protection sleeve front wall 25 has an opening 252 shaped as semi-circle extending around the needle hole 251, which is configured to allow a tool to fit through when initially assembling the components as described above.

As shown in Fig. 7B, the rotation lock sleeve 40 according to the second embodiment has an inner surface comprising four longitudinal grooves 43, wherein the four longitudinal grooves 43 each extend parallel to the central axis A. At a proximal end of the rotation lock sleeve 40, the longitudinal grooves 43 are delimited by end facets 431. Each longitudinal groove 43 is arranged between two of the rim portions 45 with respect to the circumferential direction. The needle protection sleeve 20, as shown in Fig. 7D, comprises four guide cams 24 arranged on an outer surface of the needle protection sleeve 20. Each of the four guide cams 24 is in engagement with one of the longitudinal grooves 43 of the rotation lock sleeve 40. As a resulting effect, the needle protection sleeve 20 is prevented from rotating with respect to the base and moves in a direction parallel to the central axis A from the first position to the second position. Furthermore, during retraction, the guide cams 24 abut against the end facets 431 of the longitudinal grooves 43, which then enables the needle protection sleeve 20 to drag the rotation lock sleeve in proximal direction as described above.

Furthermore, in the second embodiment the rotation lock sleeve 40 has an outer surface comprising four ridges 44 with essentially rectangular profiles, each extending parallel to the central axis A, and wherein each ridge 44 is arranged radially opposite to one of longitudinal grooves 43. As shown in Fig. 7D, the fixation ring 70 comprises four notches 71 with essentially rectangular cross-sections extending radially outwards from an inner edge 701 of the fixation ring 70. Each notch 71 is in engagement with one of the ridges 44 of the rotation lock sleeve 40, thereby linearly guiding the latter and preventing it from rotating with respect to the base 10. Instead of a rectangular shape, the ridges 44 and the corresponding notches 71 may also have any other suitable form that enables an engagement of the notches 71 with the ridges 44.

The patch-like medical according to both the first embodiment and the second embodiment discussed above further comprises a locking mechanism 60 having a locked state in which the locking mechanism 60 prevents the rotating sleeve 30 from moving when the needle protection sleeve 20 is in the first position, and an unlocked state in which the locking mechanism 60 allows the rotating sleeve 30 to carry out the helical movement causing the needle protection sleeve 20 to move from the first position to the second position.

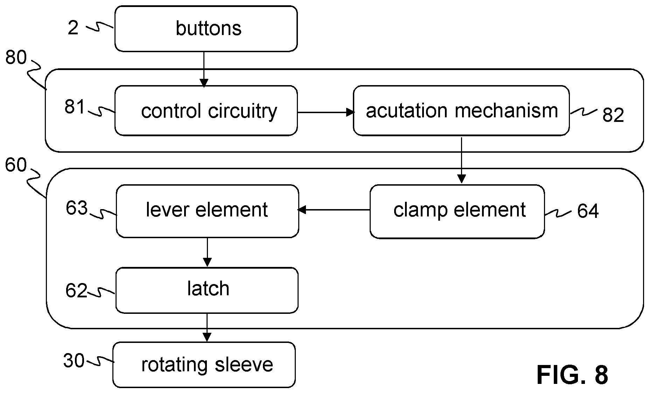

Preferably, the patch-like medical device further comprises a trigger mechanism 80 operable by the patient, for instance by pushing a button, in particular by simultaneously pushing at least two buttons 2 arranged on the patch-like medical device 1 as shown in Fig. 5, preferably by pushing three buttons, wherein the third button is arranged on a top surface of the patch-like medical device 1 (not visible in Fig. 5). Although not explicitly shown in Fig. 1, the patch-like medical device according to the first embodiment also preferably comprises an upper housing part with one or more buttons analogously to the second embodiment shown in Fig. 5. The trigger mechanism 80 is configured to switch the locking mechanism 60 from the locked state to the unlocked state. As schematically illustrated in Fig. 8, the trigger mechanism ideally comprises control circuitry 81 and an actuation mechanism 82. The locking mechanism 60 preferably comprises a latch 62, a lever element 63 connected to the latch 62 and a clamp element 64 configured to clamp the lever element 63. The latch 62 is pivotably connected to the base 10. In the locked state, the latch 62 is engaged with the rotating sleeve 30 to prevent the rotating sleeve 30 from moving. In the unlocked state, the latch 62 is disengaged from the rotating sleeve 30 to allow the rotating sleeve 30 to carry out the helical movement. The actuation mechanism is configured to mechanically actuate a clamp element 64 upon receiving an electrical signal from the control circuitry 81, thereby causing the clamp element 64 to move from a first clamp position to a second clamp position. The clamp element 64 then releases the lever element 63, allowing it to move from a first lever element position to a second lever element position as the latch pivots outwards and disengages from the rotating sleeve 30, the rotating sleeve 30 being now free to perform the helical movement causing the needle protection sleeve 20 to move from the first position to the second position to expose the needle tip 121.

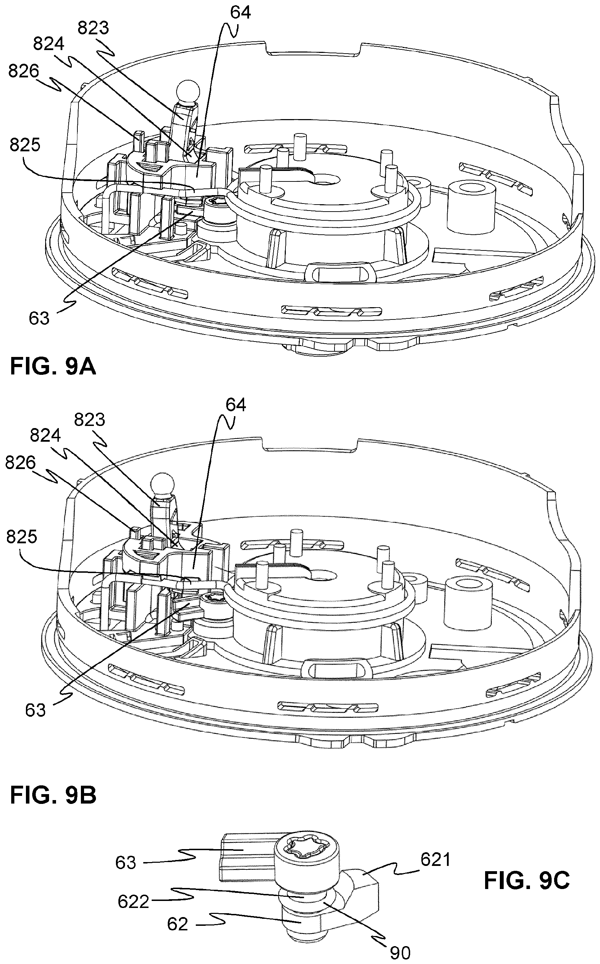

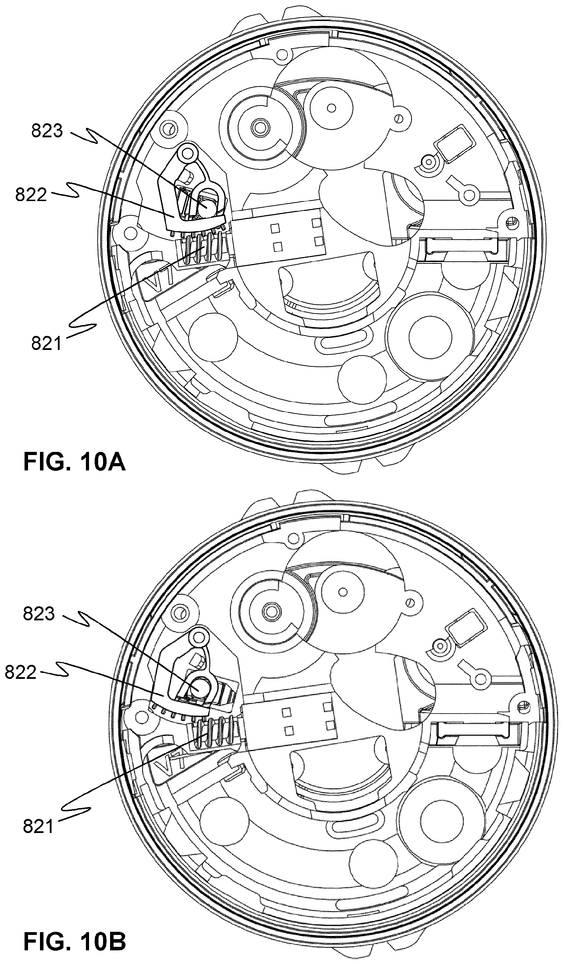

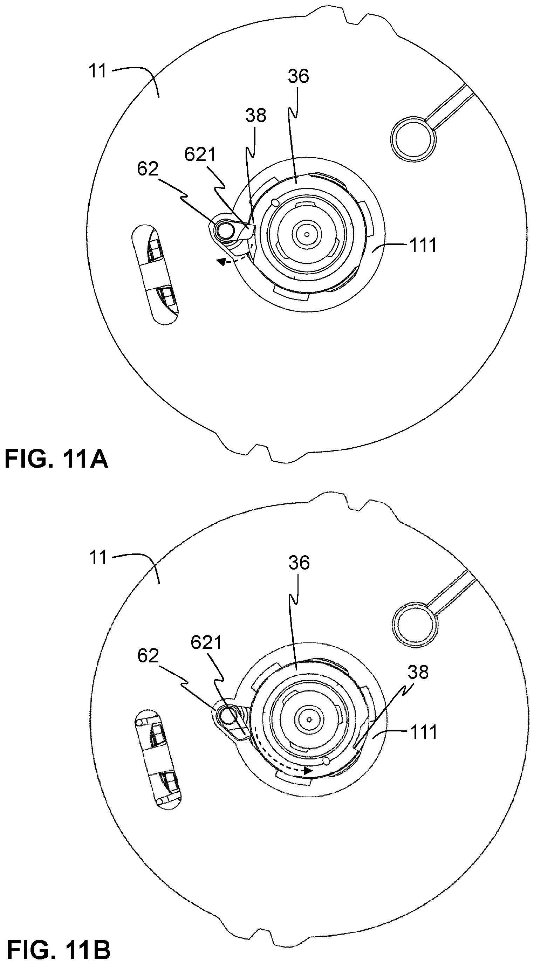

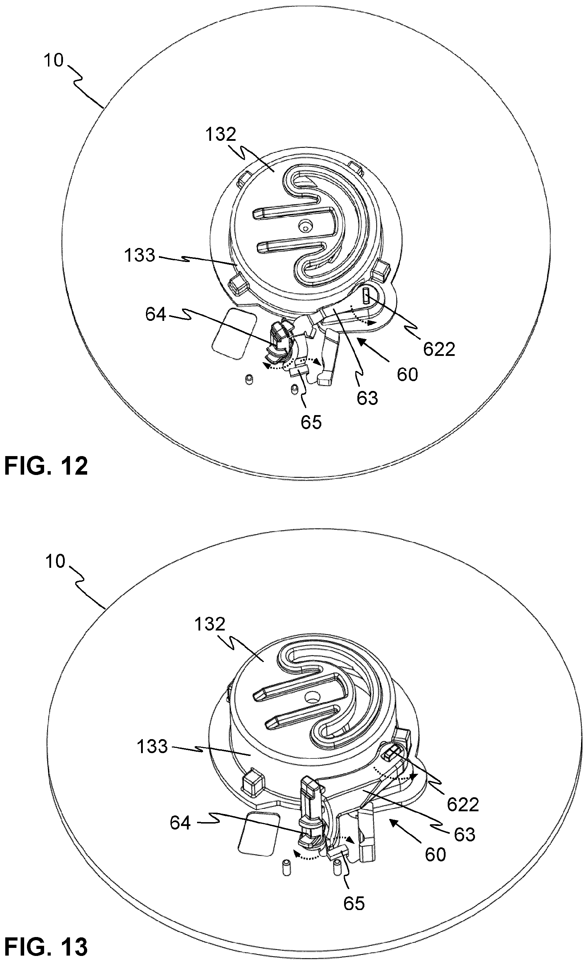

Fig. 9A and Fig. 9B illustrate a first embodiment of the locking mechanism 60 in a perspective view, wherein Fig. 9A shows the locked state and Fig. 9B shows the unlocked state. The latch 62 and the lever element 63 of this first embodiment are shown separately in an enlarged perspective view in Fig. 9C. Fig. 10A and Fig. 10B illustrate said first embodiment of the locking mechanism 60 in a top view, i.e. the viewing direction being the distal direction, wherein Fig. 10A shows the locked state, while Fig. 10B shows the unlocked state. Fig. 11A and Fig. 11B illustrate the first embodiment of the locking mechanism 60 in a bottom view, wherein Fig. 11A shows the locked state, while Fig. 11B shows the unlocked state. Fig. 12 and Fig. 13 show a second embodiment and a third embodiment of the locking mechanism 60, respectively, in a perspective view.

As visible in Fig. 11A the latch 62 comprises a tip 621 which engages with an indentation 38 located in the rim 36 of the rotating sleeve 30 (see also Fig. 3A and Fig. 7A). The latch 62 further comprises a shaft 622 extending through an opening in the base 10 parallel to the central axis A to reach the side of the base 10 opposite to the contact surface 11. To prevent any dirt from entering the patch-like medical device through said opening in the base, an O-ring 90 may arranged around the shaft to seal the opening (see Fig. 1 and Fig. 9C). On the side of the base 10 opposite to the contact surface 11, the shaft 622 of the latch 62 has an end, to which a lever element 63 is connected.

The lever element 63 may be shaped in a variety of ways; in particular, it may be an injection molded part (as shown in Fig. 9C and Fig. 13) or may be made of a sheet metal piece (as shown in Fig. 12). In all the embodiment shown here, the lever element 63 has an opening on a first end, in which the end of the shaft 622 is fixed. In the particularly advantageous first embodiment shown in Fig. 9C, said end has a star-like shaped profile which fits into the correspondingly shaped opening of the lever element 63 to enable a robust connection which is particularly suitable for enduring the torque exerted by the pre-loaded rotating sleeve 30 in the locked state, i.e. when the lever element 63 is clamped.

In the first embodiment of the locking mechanism 60 shown in Figs. 9A-11B, the actuation mechanism 82 comprises a motor-driven threaded shaft 821 and a transmission piece 822 that comprises a fork-like part (visible in Figs. 10A and 10B), wherein the threaded shaft 821 is in a gear-like engagement with a transmission piece 822. The actuation mechanism 82 further comprises a pivot arm 823 with a spherically shaped top end that is embraced by the fork-like part. Upon receiving a signal from the control circuitry, the threaded shaft 821 starts to rotate, thereby moving the transmission piece 822 from a first position to a second position. The fork-like part of the transmission piece 822 thereby drags along the spherically shaped top end of the pivot arm 823, causing the pivot arm 823 to pivot from a first pivot position to a second pivot position. The pivot arm 823 has a nose-like protrusion 824 (visible also in Figs. 2A-2C), which in the first pivot position pushes the clamp element 64 in distal direction (and thereby prevents it from moving) against a force exerted on the clamp element 64 by a resilient wire arrangement 825. As can be seen in Figs. 9A and 9B, the resilient wire arrangement 825 is partially wrapped around the sidewall 133 delimiting the recess 13 and urges the clamp element 64 to move in proximal direction from the first clamp position (where the clamp element 64 clamps the lever element 63) to the second clamp position (where the lever element 63 is released). When the pivot arm 823 is being moved to the second pivot position (Figs. 2B, 2C, 9B, 10B), the clamp element 64 moves in proximal direction, being linearly guided by a guiding structure 826 that is arranged on the base 10. As the clamp element 64 moves in proximal direction, it releases the lever element 63, which then pivots from the first lever element position to the second lever element position as the latch 62 is pushed radially outward by the rotating sleeve 30 that is urged to rotate by the pre-loaded spring 51.

In the embodiments of the lever element 63 shown in Fig. 12 and Fig. 13 the lever element 63 has a second end which is, in the first lever element position, biased in distal direction, in particular resiliently bent to abut against a stopper element 65, the stopper element 65 being attached to the base 10. In said first lever element position, which corresponds to the locked state of the locking mechanism, the lever element 63 prevents the latch 62 from disengaging from the rotating sleeve 30 by preventing rotation of the shaft 622, which is fixedly connected to the first end of the lever element 63. As indicated by the dotted arrows in Figs. 12 and 13, the lever element is movable form the first lever element position to a second lever element position in which the lever element allows the latch 62 to disengage from the rotating sleeve 30. The locking mechanism 60 further comprises a clamp element 64. The clamp element 64 is movable, here in particular rotatable, between a first clamp position in which the clamp element 64 clamps the lever element 63 in distal direction to the base 10 in the first lever element position, i.e. such that the lever element 63 abuts against the stopper element 65, and a second clamp position in which the clamp element 64 allows the lever element 63 to be released. The clamp element 64 comprises a nose, which, as the clamp element 64 is moved from the first clamp position to the second clamp position, bends the lever element 63 in proximal direction (in the embodiment shown in Fig. 12) or causes the lever element 63 to pivot (in the embodiment shown in Fig. 13) such that it does no longer abut against the stopper element 65. With the lever element 63 being free to move from the first lever element position to the second lever element position, the latch 62 is able to pivot as indicated by the dotted arrows in Fig. 11A and thus to disengage from the rim 36 of the rotating sleeve 30. The rotating sleeve 30 is then free to perform the helical movement. In cases where a spring 51 is driving said helical movement (as shown in the first and second embodiment of the patch-like medical device discussed above), the spring 51 is pre-loaded such that it exerts a force on the latch 62, in particular on the tip 621, when the needle protection sleeve 20 is in the first position and the locking mechanism 60 is in the locked state. This force urges the latch 62 to pivot radially outwards as indicated in Figs. 11A and 11B, but as long as the lever element 63 is clamped in the first lever element position in distal direction towards the base 10 by the clamp element 64, the latch 62 is prevented from pivoting by the clamped lever element 63. Once the lever element 63 is released, the latch 62 pivots outwards due to the force exerted by the spring and thereby also causes the lever element to move to the second position as indicated by the dashed arrows in Fig. 12 and Fig. 13.

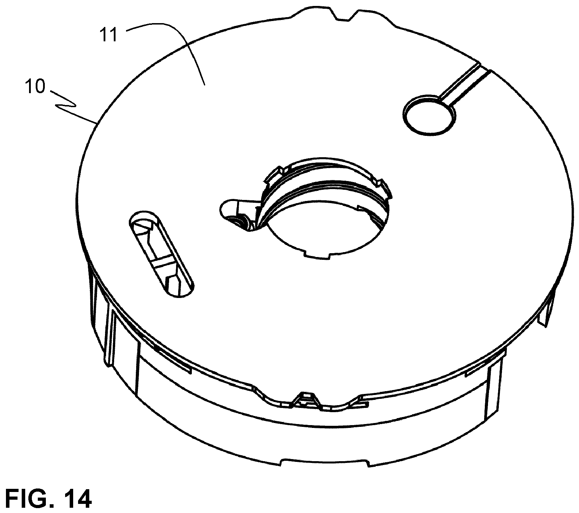

In the embodiment shown in Figs. 11A and 11B, the contact surface 11 has a recessed area 111 configured to receive the fixation ring 70 such that the fixation ring 70 is flush with the contact surface 11 when attached to the base 10 as shown in Fig. 1 and Fig. 6. As shown in Figs. 11A and 11B (where the fixation ring 70 is not shown) the latch is arranged in said recessed area 111. Once the fixation ring 70 is attached to the base 10, it at least partially covers the latch 62, such that the latch 62 does not enter into contact with the patient's skin. Alternatively, as shown in Fig. 14, the fixation ring may be formed by the base 10 itself instead of being a separate component.

Many variations of the components are conceivable without leaving the scope of the present invention and many features of the first and the second embodiment of the sleeves may be interchanged. In particular, it is possible to combine the different embodiments of the locking mechanism 60 and the trigger mechanism 80 including the actuation of the trigger mechanism 80 via one or more buttons 2 with each of the different embodiments of the sleeves shown either in Figs. 3A-3D or Figs. 7A-7D.

**LIST OF REFERENCE SIGNS**

| | | | |
|---|---|---|---|
| 1 | patch-like medical device | 35 | external thread portions |
| 2 | button | 36 | proximal rim of the rotating sleeve |
| 10 | base | | |
| 101 | protrusion of the base | 37 | rotating sleeve front wall |
| 11 | contact surface | 38 | indentation |
| 111 | recessed area | 39 | distal rim of the rotating sleeve |
| 12 | needle | | |
| 121 | needle tip | 371 | central opening |
| 13 | recess | 372 | bore |
| 131 | internal thread of the recess | 373 | arched opening |
| 132 | top wall of the recess | 40 | rotation lock sleeve |
| 133 | side wall of the recess | 41 | first bore |
| 14 | cylindrical element | 42 | guide cam |
| 141 | helical grooves | 43 | longitudinal groove |
| 20 | needle protection sleeve | 431 | end facet of the longitudinal groove |
| 21 | external thread portion (of the needle protection sleeve) | | |
| | | 44 | longitudinal ridge |
| 22 | internal thread (of the needle protection sleeve) | 45 | rim portions of the rotation lock sleeve |
| 23 | longitudinal groove | 46 | rotation lock sleeve front wall |
| 24 | guide cam | 47 | opening |
| 25 | protection sleeve contact | 471 | edge of the opening |
| | surface | 48 | notch |
| 26 | indentations | 49 | inner wall of the rotation lock sleeve |
| 251 | needle hole | | |
| 252 | opening | 491 | hook |
| 30 | rotating sleeve | 51 | spring |
| 301 | protrusion of the rotating sleeve | 511 | first end of the spring |
| | | 512 | second end of the spring |
| 31 | second bore | 60 | locking mechanism |
| 32 | thread portion | 62 | latch |
| 33 | thread portion (of the rotating sleeve) | 621 | tip |
| | | 622 | shaft |
| 34 | internal thread | 63 | lever element |
| 64 | clamp element | 821 | threaded shaft |
| 65 | stopper element | 822 | transmission piece |
| 70 | fixation ring | 823 | pivot arm |
| 701 | inner edge of the fixation ring | 824 | nose-like protrusion |
| 71 | notch | 825 | resilient wire arrangement |
| 80 | trigger mechanism | 826 | guiding structure |
| 81 | control circuitry | A | central axis |
| 82 | actuation mechanism | | |

## Claims

1. A patch-like medical device comprising:
a base (10) having a contact surface (11) configured to be directed towards a patient's skin;
a needle (12) fixedly positioned relative to the base (10), the needle (12) having a needle tip (121) which protrudes from the contact surface (11) and which is configured for piercing the patient's skin;
a needle protection sleeve (20) movable along a central axis (A) from a first position to a second position, the needle protection sleeve (20) extending beyond the needle tip (121) to prevent the needle tip (121) from piercing the patient's skin in the first position, and exposing the needle tip (121) to enable piercing the patient's skin in the second position;
a rotating sleeve (30) movable along the central axis (A), the rotating sleeve (30) being in engagement with the needle protection sleeve (20) such that a helical movement of the rotating sleeve (30) with respect to the base (10) causes the needle protection sleeve to move along the central axis (A) from the first position to the second position;
a rotation lock sleeve (40) movable along the central axis (A) while being rotationally fixed with respect to the base, the rotation lock sleeve (40) being in engagement with the needle protection sleeve (20) such that the needle protection sleeve (20) is prevented from rotating with respect to the base (10) when the needle protection sleeve (20) moves from the first position to the second position.

2. The patch-like medical device of claim 1,
wherein the rotating sleeve (30) at least partially protrudes from the contact surface (11) when the needle protection sleeve (20) is in the first position,
wherein the rotating sleeve (30) axially retracts towards the contact surface (11) along the central axis (A) during its helical movement, and wherein the needle protection sleeve (20) is configured to axially move relative to the rotating sleeve (30) along the central axis (A) during the helical movement of the rotating sleeve (30).

3. The patch-like medical device of claim 1 or 2,
wherein in the second position, the needle protection sleeve (20), the rotating sleeve (30) and the rotation lock sleeve (40) are each at least partially, preferably fully, retracted inside the patch-like medical device on a side that faces away from the contact surface (11).

4. The patch-like medical device of any one of the preceding claims,
wherein the needle protection sleeve (20) is at least partially received radially inside the rotating sleeve (30),
wherein the base (10) defines a recess (13) on a side that faces away from the contact surface, the recess being delimited by a side wall (133) in a radial direction, and
wherein the rotating sleeve (30) and the side wall (133) are in a thread-like engagement with each other.

5. The patch-like medical device of any one of claims 1-3,
wherein the rotating sleeve (30) is at least partially received radially inside the needle protection sleeve (20),
wherein the base (10) comprises a central guide element (14) extending along the central axis (A),
wherein the central guide element (14) is at least partially received radially inside the rotating sleeve (30),
and wherein the rotating sleeve (30) and the central guide element (14) are in a thread-like engagement with each other.

6. The patch-like medical device of any one of the preceding claims,
wherein the needle protection sleeve (20) and the rotating sleeve (30) are in a thread-like engagement with each other.

7. The patch-like medical device of any one of the preceding claims,
wherein one of the needle protection sleeve (20) and the rotation lock sleeve (40) comprises a longitudinal groove (23,43) extending parallel to the central axis (A) and wherein the other one of the needle protection sleeve (20) and the rotation lock sleeve (40) comprises a guide cam (24,42) configured to slide in said longitudinal groove (23,43) such that the protection sleeve (20) is prevented from rotating with respect to the base (10) when moving from the first position to the second position.

8. The patch-like medical device of any one of the preceding claims, further comprising a fixation element that is fixedly attached to the base (10) and is in engagement with the rotation lock sleeve (40) such that the rotation lock sleeve is prevented from rotating relative to the base, wherein the fixation element is preferably configured as a fixation ring (70).

9. The patch-like medical device of any one of the preceding claims, further comprising a spring (51) configured to drive the helical movement of the rotating sleeve (30), the spring (51) being pre-loaded when the needle protection sleeve (20) is in the first position, wherein the spring (51) at least partially relaxes when the needle protection sleeve (20) moves from the first position to the second position.

10. The patch-like medical device of any one of the preceding claims, further comprising a locking mechanism (60) having a locked state in which the locking mechanism (60) prevents the rotating sleeve (30) from moving when the needle protection sleeve (20) is in the first position, and an unlocked state in which the locking mechanism (60) allows the rotating sleeve (30) to carry out the helical movement so as to cause the needle protection sleeve (20) to move from the first position to the second position.

11. The patch-like medical device of claim 10, further comprising a trigger mechanism (80) operable by the patient, wherein the trigger mechanism (80) is configured to switch the locking mechanism (60) from the locked state to the unlocked state.

12. The patch-like medical device of claim 10 or 11, wherein the locking mechanism (60) comprises a latch (62) pivotably connected to the base (10), the latch (62) being engaged with the rotating sleeve (30) in the locked state to prevent the rotating sleeve (30) from moving and disengaged from the rotating sleeve (30) in the unlocked state to allow the rotating sleeve (30) to carry out the helical movement.

13. The patch-like medical device of claim 12, wherein the locking mechanism (60) comprises a lever element (63) being connected to the latch (62) for facilitating control of the latch (62), wherein the lever element (63) is movable between a first lever element position in which the lever element (63) prevents the latch (62) from disengaging from the rotating sleeve (30), and a second lever element position in which the lever element allows the latch (62) to disengage from the rotating sleeve (30).

14. The patch-like medical device of claim 13, wherein the locking mechanism (60) further comprises a clamp element (64) configured for clamping the lever element (63) to prevent inadvertent motion of the lever element (63) and the latch (62), wherein the clamp element (64) is movable between a first clamp position in which the clamp element (64) fixes the lever element (63) with respect to the base (10) in the first lever element position, and a second clamp position in which the clamp element (64) releases the lever element (63) and allows the lever element (63) to move from the first lever element position to the second lever element position.

15. The patch-like medical device of claim 14, wherein the trigger mechanism comprises control circuitry (81) and an actuation mechanism (82) configured to mechanically actuate the clamp element (64) upon receiving an electrical signal from the control circuitry (81), causing the clamp element (64) to move from the first clamp position to the second clamp position so as to release the lever element (63).
